Europäisches Patentamt

European Patent Office

Office européen des brevets

(19)

(11) Veröffentlichungsnummer: **0 302 001**
**A1**

(12) **EUROPÄISCHE PATENTANMELDUNG**

(21) Anmeldenummer: 88730143.0

(22) Anmeldetag: 01.07.88

(51) Int. Cl.⁴: **A 61 M 25/00**
A 61 B 17/52

(30) Priorität: 02.07.87 DE 8709240

(43) Veröffentlichungstag der Anmeldung:
01.02.89  Patentblatt  89/05

(84) Benannte Vertragsstaaten: DE FR GB IT

(71) Anmelder: **Effner GmbH**
**Alt-Lankwitz 102**
**D-1000 Berlin 46  (DE)**

(72) Erfinder: **Anapliotis, Emmanuel**
**Kiebitzweg 5**
**D-1000 Berlin 33  (DE)**

(74) Vertreter: **Christiansen, Henning, Dipl.-Ing.**
**Patentanwalt CHRISTIANSEN Pacelliallee 43/45**
**D-1000 Berlin 33  (DE)**

(54) **Magnetsonde mit Katheter.**

(57) Magnetsonde mit einem Katheter zum Einführen in den Magen-Darm-Trakt, wobei ein Magnet (14) im Bereich des distalen Endes des Katheters (1) in einer Kapsel (13) senkrecht zu einer der Wandungen der Kapsel (13) bis zum Anschlag an deren Innenseite (15) verschieblich gelagert ist, wobei der Magnet (14) mit einem sich zum proximalen Ende des Katheters (1) hin erstreckenden Betätigungsmechanismus (4) verbunden ist.

Fig. 3

EP 0 302 001 A1

**Beschreibung**

## Magnetsonde mit Katheter

Die Erfindung betrifft eine Magnetsonde der im Oberbegriff des Anspruchs 1 angegebenen Art.

Das Verschlucken von metallhaltigen Fremdkörpern (FK) ist ein relativ häufiges Ereignis im pädiatrischen Patienten gut. Insbesondere die - Knopfbatterien - können für lebensbedrohende Zustände Anlaß geben. Die Knopfbatterien enthalten Quecksilber, ein für den menschlichen Stoffwechsel toxisches Substrat.

Hierbei kann es zu lebensbedrohenden Zuständen für den Patienten kommen. Bedingt durch den hohen technischen Entwicklungsgrad werden immer mehr Objekte produziert, die leicht verschluckt werden können. Als besonders gefährlich sind die Quecksilber enthaltenden Knopfbatterien einzustufen. Neuere Berichte weisen darauf hin, daß Knopfbaterien einer sofortigen therapeutischen Entfernung zuzuführen sind. Bei bekannten Magnetsonden, welche einen an einem Leitinstrument befestigten Magneten aufweisen, besteht der Nachteil, daß eine Korrektur der Postion des Fremdkörpers in bezug auf den Magneten nicht möglich ist.

Der Erfindung liegt die Aufgabe zugrunde, bei einer Magnetsonde der eingangs genannten Gattung die Möglichkeit zu schaffen, den Kontakt zwischen Magnet und zu entfernendem Fremdkörper nach Bedarf auch zu unterbrechen, um gegebenenfalls eine noch günstigere Position von Sonde und Fremdkörper zum Entfernen des letzteren zu finden.

Diese Aufgabe wird mit den kennzeichnenden Merkmalen des Anspruchs 1 gelöst.

Die erfindungsgemäße Sonde bietet eine vorteilhafte Möglichkeit, alle radiologisch gesicherten metallhaltigen Fremdkörperingestionen, lokalisierbar im Ösophagus, Magen bis zum Duodenum, zu entfernen. Unter Durchleuchtungskon trolle ist der schattengebende Fremdkörper jederzeit darstellbar und optimal für die Magnet-Extraktion positionierbar.

Im Gegensatz zur Endoskopie ist die Erfolgsrate der beschriebenen Technik, insbesondere bei Magenlokalisationen, trotz Flüssigkeits- und Speiseresten bei den Patienten bei nahezu 100%. Diese Technik ist für alle schattengebenden magnetischen Fremdkörper anwendbar, (z.B. Knopfbatterien, Münzen, Nadeln etc.).

Die Extraktionen werden mittels eines Vacomax 200-Magneten auf der Basis SmCo durchgeführt. Die Remanenz des axialen Stabmagneten beträgt 0,9 Tesla. Der axiale Stabmagnet mißt 0,5 cm im Querdurchmesser und 2 cm im Längsdurchmeser.

Die Sonde wird ohne Narkose im Sitzen oroösophageal eingeführt. Am liegenden Patienten wird unter Durchleuchtungskontrolle der Fremdkörper detektiert, so daß ein optimaler Raum zum Kontaktieren mit dem Magneten ausgewählt werden kann. Der ganze Vorgang kann dabei mit einem Durchleuchtungsbildaufzeichnungsgerät dokumentiert werden.

In der Gruppe mit Knopfbatterieningestion gelang es mit der erfindungsgemäßen Sonde, bei einer Zahl von fünf Patienten diese in jedem Fall zu entfernen. Die Röntgenlagedefinition war in allen fünf Fällen distal der Kardia. Die Durchleuchtungszeit betrug max. 2,5 min.

In einer Gruppe mit verschiedenen metallhaltigen Fremdkörperingestionen befanden sich zwei Fremdkörper proximal der Kardia und neun Fremdkörper im Magen oder Duodenum. Alle neun Fremdkörper-Extraktionen distal der Kardia verliefen erfolgreich.

Besonders vorteilhaft bei der erfindungsgemäßen Magnetsonde ist, daß der Betätigungsmechanismus aus einem im Inneren des Katheters geführten Mandrin besteht, der am proximalen Ende des Katheters aus diesem herausragt und an dessen distalem Ende mit dem Magneten verbunden ist, so daß eine direkte Betätigungsverbindung ohne komplizierte Übertragungsmechanismen möglich ist. Wenn der Mandrin für Röntgenstrahlen undurchlässig ist, läßt sich der Weg der Sonde ohne Schwierigkeiten verfolgen. Dadurch, daß mindestens die Wandung der Kapsel, zu der der Magnet in senkrechter Richtung verschieblich gelagert ist, aus einem ferromagnetischen Werkstoff besteht, läßt sich die mechanische Kraftwirkung des Magneten lokal beeinflussen. Wenn der als zylindrischer Stabmagnet ausgebildete Magnet in Längsrichtung magnetisiert und in der zylindrisch ausgebildeten Kapsel axial verschieblich geführt ist, ergibt sich eine optimal verkleinerte Bauform. Wenn mindestens Teile der Seitenwandungen der Kapsel, denen der Magnet nur in einer seiner Endpositionen benachbart ist, für Röntgenstrahlen durchlässig ausgebildet sind, ist auch die Position des Magneten röntgenologisch erfaßbar. Dadurch, daß bevorzugt im Bereich der Verbindung zwischen Kapsel und distalem Ende des Katheters eine Öffnung zum Inneren des Katheters hin vorgesehen ist, kann von proximal Kontrastmittel eingebracht werden, wobei der Mandrin an seinem proximalen Ende ein Griffstück aufweist, welches die entsprechende Öffnung des Katheters bei eingeschobenem Griff stück dichtend verschließt und daß eine seitliche Öffnung mit einem Anschluß für eine entsprechende Schlauchverbindung vorgesehen ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 das proximale Ende eines Katheters als Ausführungsbeispiel der Erfindung in vergrößerter Darstellung,

Figur 2 das distale Ende des Katheters gemäß Figur 1 sowie

Figur 3 einen Schnitt durch das distale Ende des Katheters.

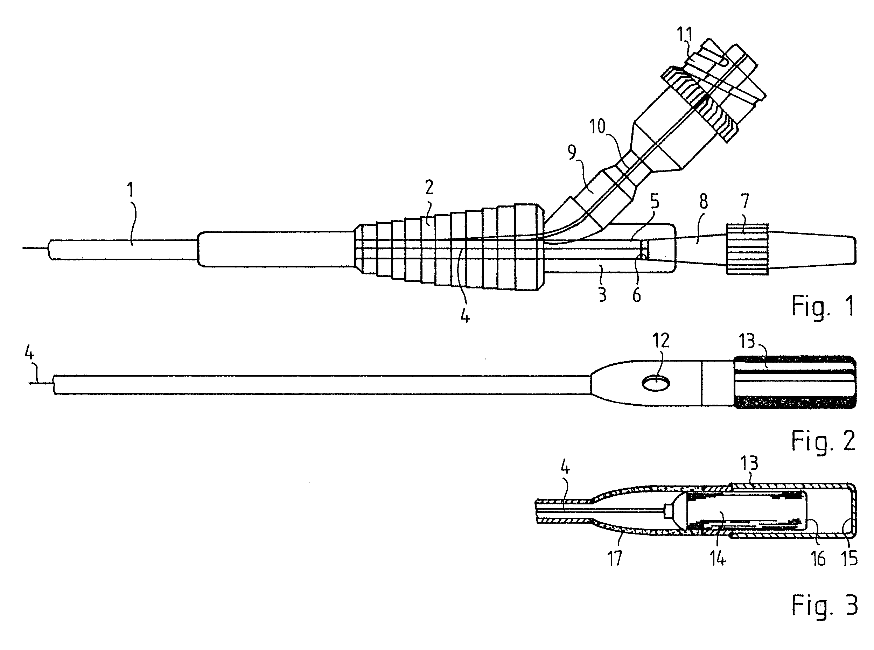

Der in Figur 1 dargestellte Katheter 1 weist zu seinem proximalen Ende hin einen sich konisch erweiternden Bereich als Griffstück 2 auf, welches zur Steuerung in der einen Hand geführt wird. Das Griffstück 2 und ein sich an diesen anschließender zylindrischer Bereich 3 sind transparent ausgebildet, so daß der Verlauf eines Mandrins 4 durchscheint.

Der Mandrin 4 wird in einem Innenkanal 5 geführt, welcher distal in einer konischen Erweiterung 6 endet. Der Mandrin 4 ist seinerseits am proximalen Ende mit einem Griff 7 versehen, der sich in einem Bereich 8 konisch verjüngt, wobei dieser Bereich mit dem Innenkonus 6 des Bereichs 3 einen dichtenden Sitz bildet, mit dem das einen Durchlaß für den Mandrin aufweisende Katheterende verschlossen werden kann. Dieser Sitz ist auch für den hier dargestellten Anwendungsbereich druckfest, da der aus einem flexiblen Material hergestellte Katheter eine kraftschlüssige Verbindung erzeugt, wenn das Griffteil 7 entsprechend einem Stopfen mit einem gewissen Druck in die Öffnung eingetrieben gehalten wird. Dabei kehren sich die Verhältnisse von Stopfen und zu verschließender Öffnung in soweit gegenüber den üblichen Verhältnissen um, als hier der Stopfen starr und die zu verschließende Öffnung elastisch ausgebildet ist.

Ein Abzweig 9 weist einen inneren Kanal 10 und einen sogenannten Luer-Lock-Schraubanschluß 11 auf, mit dem ein Kontrastmittel unter Druck in das Innere des Katheters eingeleitet werden kann, wobei in diesem Fall das Ende 3 mit dem Griffstück 7 zu verschließen ist. Der Kanal 10 steht mit dem Inneren des Katheters 1 in Verbindung, der einen gewissen Spielraum zum Mandrin 4 hin für den Durchtritt des Kontrastmittels aufweist.

Das proximale Ende des Katheters 4, welches in Figur 2 dargestellt ist, weist eine Öffnung 12 auf, aus der das Kontrastmittel seitlich austreten und somit gezielt appliziert werden kann.

Das distale Ende des Mandrins endet in einer Kammer 13, welche als stirnseitig geschlossener zylindrischer Hohlzylinder ausgebildet ist. In diesem Hohlzylinder 13 ist - wie die Schnittdarstellung gemäß Figur 3 zeigt - ein zylindrischer Stabmagnet 14 in axialer Richtung beweglich vorgesehen, der mit dem Ende des Mandrins 4 fest verbunden ist. Der Stabmagnet 14 ist 2 cm lang und weist einen Durchmesser von 0,5 cm auf. Er besteht aus einer Legierung auf der Basis Neodyn-Eisen-Bor, bekannt unter der Bezeichnung "Vacodyn".

Das Ende der Kapsel 13 weist in der Länge des Stabmagneten eine ferromagnetische Umkleidung auf, welche den Stabmagneten ausreichend dicht umschließt, um einen möglichst geringen Luftspalt für die Feldlinien zu bilden. Dabei kommt es insbesondere auf die Übereinstimmung der planen Innenfläche 15 der Kammer 13 und der Stirnfläche 16 des Magneten an. Das Material der Umkleidung ist relativ dünnwandig und weist eine kleine Remanenz auf, so daß die magnetischen Eigenschaften des distalen Endes der Sonde von der Position des Stabmagneten 14 abhängen. Der an den magnetisch leitenden Bereich 13 nach proximal anschließende Bereich 17 des Katheters besteht aus röntgentransparentem Kunststoff, so daß hier eine "magnetische Isolation" vorliegt. Auf diese Weise lassen sich die magnetischen Eigenschaften des distalen Katheterendes durch die Position des Stabmagneten 14 bestimmen.

Da die Kammer 13 für Röntgenstrahlen undurchlässig ist, was auch für den Stabmagneten 14 und den Mandrin 4 gilt, läßt sich sowohl der Verlauf des Mandrins als auch die Position des Magneten (sowie des zu entfernenden metallhaltigen Fremdkörpers) mit einem Röntgensichtgerät jederzeit kontrollieren, da der Durchmesser des Mandrins und des Magneten 14 bzw. der Kammer 13 sich deutlich unterscheiden.

Der Mandrin 4 weist einen Durchmesser auf, der ausreichend ist, um dem Verlauf des Katheters zu folgen und dessen Weg zu kontrollieren. Die Länge des aus den Elementen 13 und 14 gebildeten für Röntgenstrahlen undurchsichtigen Komplexes - bezogen auf die axiale Richtung - hängt von der Position des Magneten innerhalb oder außerhalb der Kammer 13 ab.

Es ist ersichtlich, daß mit der beschriebenen Sonde ein verschluckter metallhaltiger Gegenstand mit magnetischen Eigenschaften durch Einführung des distalen Sondenendes gezielt angesteuert werden kann. Der mechanische Kraftschluß zwischen Sondenende und Fremdkörper kann dabei durch (gegebenenfalls wiederholtes) Lösen des magnetischen Kraftschlusses so manipuliert werden, daß sich der Fremdkörper in einer zum Extrahieren günstigen Position befindet. Zum Lösen des Kraftschlusses braucht dabei jeweils nur das Griffteil 7 gegenüber dem Haltestück 2 axial verschoben zu werden, indem der Griff herausgezogen wird. Damit wird der Magnet aus dem magnetisch leitenden Bereich der Kammer 13 entfernt, so daß sich der Fremdkörper löst und nach Änderung der Lage des distalen Endes der Sonde erneut in magnetischen Kontakt mit der Kammer 13 gebracht werden, wobei der Magnet 14 durch Eindrücken des Griffes 7 wieder zum Ende vorgeschoben wird. Obgleich die Position des Magneten durch die Stellung des Griffes 7 bereits eindeutig bestimmt ist, ermöglicht die Röntgenkontrolle eine zusätzliche Sicherheit. Die Sonde gestattet es somit auch, Fremdkörper komplizierter Form in eine solche Lage zur Sonde zu bringen, daß ein Entfernen unter den günstigsten Umständen möglich ist. Dazu gehört beispielsweise die Be rücksichtigung einer Verjüngung des zu extrahierenden Gegenstandes oder dessen Massenverteilung, um einen möglichst sicheren Kraftschluß mit dem Magneten zu erzielen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Magnetsonde mit einem Katheter zum Einführen in den Magen-Darm-Trakt,
**dadurch gekennzeichnet,**
daß ein Magnet (14) im Bereich des distalen Endes des Katheters (1) in einer Kapsel (13) senkrecht zu einer der Wandungen der Kapsel bis zum Anschlag an deren Innenseite verschieblich gelagert ist, wobei der Magnet mit einem sich zum proximalen Ende des Katheters hin erstreckenden Betätigungsmechanismus (4, 7) verbunden ist.

2. Magnetsonde nach Anspruch 1, **dadurch gekennzeichnet,** daß der Betätigungsmechanismus aus einem im Inneren des Katheters geführten Mandrin (4) oder Zugdraht besteht, der am proximalen Ende des Katheters (1) aus diesem herausragt und an dessen distalem Ende mit dem Magneten verbunden ist.

3. Magnetsonde nach einem der vorangehenden Ansprüche, **dadurch gekennzeichnet,** daß der Mandrin (4) oder Zugdraht für Röntgenstrahlen undurchlässig ist.

4. Magnetsonde nach einem der vorangehenden Ansprüche, **dadurch gekennzeichnet,** daß minde stens die Wandung der Kapsel (13), zu der der Magnet in senkrechter Richtung verschieblich gelagert ist, aus einem ferromagnetischen Werkstoff besteht.

5. Magnetsonde nach einem der vorangehenden Ansprüche, **dadurch gekennzeichnet,** daß der als Magnet (14) als zylindrischer Stabmagnet ausgebildet ist, und in der zylindrisch ausgebildeten Kapsel axial verschieblich geführt ist.

6. Magnetsonde nach einem der vorangehenden Ansprüche, **dadurch gekennzeichnet,** daß der Magnet (14) in Längsrichtung des Katheters magnetisiert ist.

7. Magnetsonde nach einem der vorangehenden Ansprüche, **dadurch gekennzeichnet,** daß mindestens Teile der Seitenwandungen (17) der Kapsel, denen der Magnet (14) nur in einer seiner Endpositionen benachbart ist, für Röntgenstrahlen durchlässig ausgebildet sind.

8. Magnetsonde nach einem der vorangehenden Ansprüche, **dadurch gekennzeichnet,** daß am proximalen Ende des Katheters ein seitlicher, insbesondere Luer-Lock-, Anschluß (9) mit einer zum Inneren des Katheters führenden Bohrung vorgesehen ist.

9. Magnetsonde nach einem der vorangehenden Ansprüche, **dadurch gekennzeichnet,** daß im Bereich der Verbindung zwischen Kapsel und distalem Ende des Katheters eine zum Inneren des Katheters (1) hinführende Öffnung (12) vorgesehen ist.

10. Magnetsonde nach Anspruch 7, **dadurch gekennzeichnet,** daß die Durchlaßöffnung für den Mandrin (4) oder Zugdraht am proximalen Ende des Katheters - insbesondere durch ein in diese Öffnung eingeschiebbares Griffstück (7) des Mandrins oder Zugdrahts - dichtend verschließbar ist.

Fig. 1

Fig. 2

Fig. 3

0302001

| | EINSCHLÄGIGE DOKUMENTE | | EP 88730143.0 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl.4)** |
| X | US - A - 3 332 425 (F.E.LUBORSKY et al.) | 1-5,7 | A 61 M 25/00 A 61 B 17/52 |
| Y | * Fig. 5; Spalte 1, Zeilen 12-21,60-63; Spalte 2, Zeilen 4-15,20-24; Spalte 3, Zeile 40 - Spalte 4, Zeile 6 * | 6,8,9 | |
| A | | 10 | |
| | -- | | |
| Y | US - A - 2 517 325 (A.H.LAMB) | 6 | |
| | * Fig. 1 * | | |
| | -- | | |
| Y | US - A - 2 706 979 (F.J.WALLACE) | 8,9 | |
| | * Fig. 1,2; Spalte 2, Zeilen 49-65; Spalte 2, Zeile 73 - Spalte 3, Zeilen 1,9-15 * | | |
| | ---- | | |
| | | | **RECHERCHIERTE SACHGEBIETE (Int. Cl.4)** |
| | | | A 61 B 5/00 A 61 B 17/00 A 61 M 23/00 A 61 M 25/00 A 61 N 1/00 |

Der vorliegende Recherchenbericht wurde für alle Patentansprüche erstellt.

| Recherchenort | Abschlußdatum der Recherche | Prüfer |
|---|---|---|
| WIEN | 22-11-1988 | LUDWIG |